# EUROPEAN PATENT APPLICATION

(11) **EP 4 491 609 A1**
(43) Date of publication of application: **15.01.2025**
(21) Application number: 23860783.2
(22) Date of filing: 24.08.2023
(51) Int. Cl.: C07C 51/43, C07C 51/44, C07C 57/04

(54) **METHOD FOR PREPARATION OF HIGH PURITY (METH)ACRYLIC ACID**

(30) Priority: 30.08.2022 KR 20220109514; 16.08.2023 KR 20230107059
(71) Applicant: LG Chem, Ltd., Yeongdeungpo-gu Seoul 07336 (KR)
(72) Inventor: YOO, Sung Jin, Daejeon 34122 (KR); JANG, Kyung Soo, Daejeon 34122 (KR); LEE, Sung Kyu, Daejeon 34122 (KR)
(74) Representative: Goddar, Heinz J.
(86) International application number: PCT/KR2023/012563
(87) International publication number: WO 2024/049106

(57) **Abstract**

Provided is a method for preparation of (meth)acrylic acid including: bringing a mixed gas including (meth)acrylic acid into contact with water in an absorption column to obtain first and second (meth)acrylic acid aqueous solutions; discharging the first (meth)acrylic acid aqueous solution from a lower portion of the absorption column and supplying the solution to a crystallizer, and discharging the second (meth)acrylic acid aqueous solution from a side portion of the absorption column; supplying the second (meth)acrylic acid aqueous solution to a water separation column to obtain a distillate including (meth)acrylic acid and high-boiling point by-products; supplying the distillate to a high-boiling point by-product separation column and supplying an upper discharge stream of the high-boiling point by-product separation column including (meth)acrylic acid to the crystallizer; and obtaining purified (meth)acrylic acid in the crystallizer and circulating a part of a mother liquor recovered in the crystallizer to the absorption column and the rest to the water separation column.

## Description

### [Technical Field]

### CROSS-REFERENCE TO RELATED APPLICATIONS

The present application claims the benefit of priorities to Korean Patent Application No. 10-2022-0109514, filed on August 30, 2022, and Korean Patent Application No. 10-2023-0107059, filed on August 16, 2023, the entire contents of which are incorporated herein as a part of the specification.

### Technical Field

The present invention relates to a method for preparation of high-purity (meth)acrylic acid.

### [Background Art]

(Meth)acrylic acid is generally prepared by a method of subjecting a compound such as propane, propylene, and (meth)acrolein to a gas phase oxidation reaction in the presence of a catalyst. For example, propane, propylene, and the like are converted through (meth)acrolein into (meth)acrylic acid by a gas phase oxidation reaction in the presence of an appropriate catalyst in a reactor, and a mixed gas including (meth)acrylic acid, unreacted propane or propylene, (meth)acrolein, inert gas, carbon dioxide, water vapor, and various organic by-product by the reaction (such as acetic acid, low-boiling point by-products, and high-boiling point by-products) is obtained in a latter stage of the reactor.

The (meth)acrylic acid-containing mixed gas is brought into contact with an absorption solvent such as water in an absorption column to be recovered as a (meth)acrylic acid aqueous solution. Further, as a subsequent process for recovering the (meth)acrylic acid included in the (meth)acrylic acid aqueous solution, it is common to involve processes such as extraction, distillation, and purification. In order to improve recovery efficiency of the (meth)acrylic acid, various methods of adjusting process conditions, process order, or the like have been suggested.

However, since the absorption solvent such as water used in the absorption column has a high specific heat, significantly high energy usage is demanded in separating by-products from the (meth)acrylic acid aqueous solution including water through a process such as distillation. Meanwhile, when the subsequent process is simplified and shortened for reducing the energy usage, the energy usage may be decreased, but it is difficult to obtain high-purity (meth)acrylic acid.

Furthermore, acetic acid and high-boiling point by-products which are main by-products of a preparation process of (meth)acrylic acid are separated and removed so that they do not accumulate in the system, and in this process, loss of (meth)acrylic acid, which is the desired product, is incurred.

Therefore, it is urgent to introduce a technology which may reduce the energy usage throughout the entire process, while also minimizing loss of high-purity (meth)acrylic acid from a (meth)acrylic acid aqueous solution.

### [Disclosure]

### [Technical Problem]

In order to solve the problems mentioned in the Background Art, an object of the present invention is to provide a method for recovering (meth)acrylic acid, which may further reduce energy usage in a purification process, while securing high-purity (meth)acrylic acid with a high recovery rate.

### [Technical Solution]

In one general aspect, a method for preparation of (meth)acrylic acid includes: bringing a mixed gas including (meth)acrylic acid into contact with water in an absorption column to obtain first and second (meth)acrylic acid aqueous solutions; discharging the first (meth)acrylic acid aqueous solution from a lower portion of the absorption column and supplying the solution to a crystallizer, and discharging the second (meth)acrylic acid aqueous solution from a side portion of the absorption column; supplying the second (meth)acrylic acid aqueous solution to a water separation column to obtain a distillate including (meth)acrylic acid and high-boiling point by-products; supplying the distillate to a high-boiling point by-product separation column and supplying an upper discharge stream of the high-boiling point by-product separation column including (meth)acrylic acid to the crystallizer; and obtaining purified (meth)acrylic acid in the crystallizer and circulating a part of a mother liquor recovered in the crystallizer to the absorption column and the rest to the water separation column.

### [Advantageous Effects]

According to the method for preparation of (meth)acrylic acid according to the present invention, an amount of water introduced to an absorption column for purifying (meth) acrylic acid is minimized and a part of (meth)acrylic acid aqueous solution discharged from the absorption column is directly supplied to a crystallizer without performing a distillation process, thereby reducing energy usage consumed in the entire process.

In addition, a water separation column and a layer separator may be provided after the absorption column to separate and remove acetic acid as a by-product, and thus, the loss of (meth)acrylic acid from an upper portion of the absorption column may be decreased as compared with the case in which all acetic acid is discharged from the upper portion of the absorption column.

Furthermore, the high-boiling point by-products may be separated after the water separation column to prevent accumulation of the high-boiling point by-products in the system and an upper discharge stream of the high-boiling point by-products is supplied again to the crystallization, thereby further decreasing the loss of (meth)acrylic acid.

### [Description of Drawings]

FIG. 1 is a process flow chart showing a method for preparation of (meth)acrylic acid according to an exemplary embodiment of the present invention.
FIGS. 2 to 4 are process flow charts showing methods for preparation of (meth)acrylic acid according to the comparative examples of the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

The term "stream" in the present invention may refer to a fluid flow in a process, or may refer to a fluid itself flowing in a pipe. Specifically, the stream may refer to both a fluid itself flowing in a pipe connecting each device and a fluid flow. In addition, the fluid may refer to a gas or liquid, and a case in which a solid substance is included in the fluid is not excluded.

Meanwhile, in the devices such as an absorption column, a degassing column, a distillation column or a distillation column, and a crystallizer, unless otherwise particularly stated, a "lower portion" of the device refers to a point at a height of 95% to 100% from top to bottom of the device, specifically a lowest part (bottom). Likewise, unless otherwise particularly stated, an "upper portion" of the device refers to a point at a height of 0% to 5% from top to bottom of the device, specifically a highest part (top).

Hereinafter, the present invention will be described in more detail for better understanding of the present invention.

The method for preparation of (meth)acrylic acid according to an exemplary embodiment of the present invention may include: bringing a mixed gas including (meth)acrylic acid into contact with water in an absorption column to obtain first and second (meth)acrylic acid aqueous solutions; discharging the first (meth)acrylic acid aqueous solution from a lower portion of the absorption column and supplying the solution to a crystallizer, and discharging the second (meth)acrylic acid aqueous solution from a side portion of the absorption column; supplying the second (meth)acrylic acid aqueous solution to a water separation column to obtain a distillate including (meth)acrylic acid and high-boiling point by-products; supplying the distillate to a high-boiling point by-product separation column and supplying an upper discharge stream of the high-boiling point by-product separation column including (meth)acrylic acid to the crystallizer; and obtaining purified (meth)acrylic acid in the crystallizer and circulating a part of a mother liquor recovered in the crystallizer to the absorption column and the rest to the water separation column.

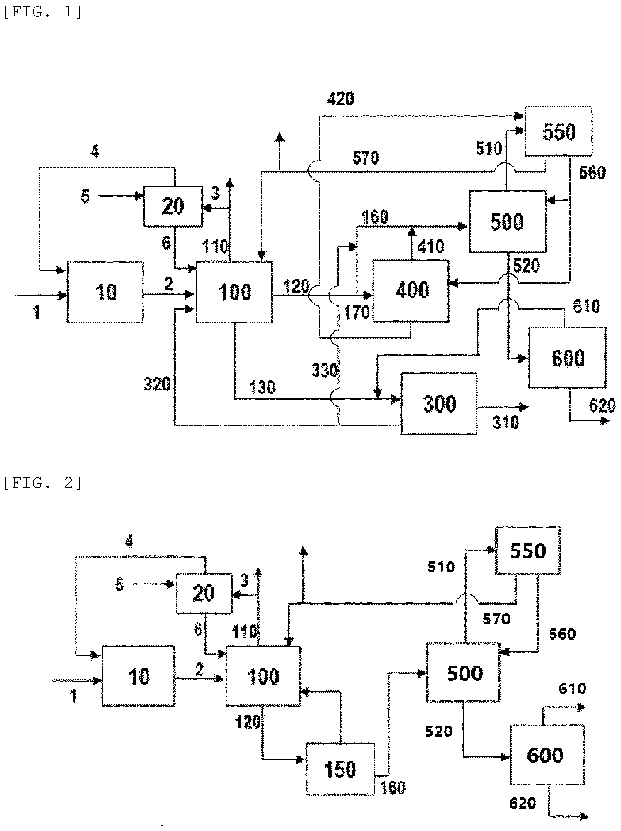

Hereinafter, each process which may be included in the exemplary embodiment of the present invention will be described, with reference to FIG. 1 and the like.

First, the method for preparation of (meth)acrylic acid according to an exemplary embodiment of the present invention may include bringing a mixed gas including (meth)acrylic acid into contact with water in an absorption column to obtain first and second (meth)acrylic acid aqueous solutions. Herein, the mixed gas including (meth)acrylic acid is a concept which collectively refers to gas phase components discharged from a reactor 10 which produces (meth)acrylic acid by a gas phase oxidation reaction. Specifically, the mixed gas may include (meth)acrylic acid, an unreacted raw material compound, (meth)acrolein, inert gas, carbon monoxide, carbon dioxide, water vapor, various organic by-products (acetic acid, low-boiling point by-products, high-boiling point by-product, etc.), and the like. Herein, a "low-boiling point by-product" (light ends) or a "high-boiling point by-product" (heavies) is a kind of by-product which may be produced in preparation and recovery processes of the target (meth)acrylic acid, and may be a compound having a higher or lower molecular weight than the (meth)acrylic acid.

Specifically, the mixed gas including (meth)acrylic acid may be prepared as follows.

First, a reaction gas including gas containing oxygen and a raw material compound is supplied to a reactor 10 provided with a catalyst through a reaction gas supply line 1, and is subjected to a gas phase oxidation reaction in the presence of a catalyst in the reactor 10 to obtain the mixed gas including (meth)acrylic acid.

Herein, the gas containing oxygen may be air. The raw material compound may be one or more compounds selected from the group consisting of propane, propylene, butane, 1-butylene, t-butylene, and (meth)acrolein, and specifically, the raw material compound may include propylene. Meanwhile, the reaction gas supplied to the reactor 10 may further include a recirculation gas which is recovered from an upper portion of the absorption column 100 and recirculated.

The recirculation gas may be derived from the upper portion of the absorption column 100 described later. That is, the mixed gas comes into contact with water which is an absorption solvent in the absorption column 100, and a non-condensable gas which is not dissolved in water may be discharged as an upper discharge stream 110 of the absorption column 100. The non-condensable gas may include impurities such as acetic acid, inert gas, an unreacted raw material compound, and a minimum content of (meth)acrylic acid. The recirculation gas may be supplied to the reactor 10 so that it may be used in the gas phase oxidation reaction for preparing (meth)acrylic acid which proceeds in the reactor.

That is, when acetic acid is discharged from the upper portion of the absorption column 100, as an amount of discharged acetic acid is increased, a content of (meth)acrylic acid discharged from the upper portion of the absorption column 100 tends to be increased. This means loss of (meth)acrylic acid. As described later, according to the present invention, since acetic acid in the system may be further separated and removed by a water separation column and a layer separator after the absorption column, the acetic acid in the system does not need to be discharged forcefully to the upper discharge stream 110 of the absorption column, and specifically, the acetic acid in an amount sufficient to minimize a content of (meth)acrylic acid in the upper discharge stream 110 of the absorption column may be discharged as the upper discharge stream 110. Thus, the content of (meth)acrylic acid which is discharged from the upper portion of the absorption column 100 and lost may be minimized.

Meanwhile, a part 3 of the upper discharge stream 110 of the absorption column may be supplied to a cooling tower 20, and the rest may be supplied to a waste gas incinerator and discarded.

The cooling tower 20 is provided with a water supply line 5 in the upper portion, and water used as an absorption solvent in the absorption column may be supplied from the water supply line 5 into the cooling tower 20. In the cooling tower 20, water may come into contact with the non-condensable gas included in the part 3 of the upper discharge stream 110 of the absorption column. As described above, the non-condensable gas may include acetic acid and a minimal amount of (meth)acrylic acid, and these components are dissolved in the water, which may be discharged in an aqueous solution form as a lower discharge stream of the cooling tower 20. Thereafter, a lower discharge stream 6 of the cooling tower 20 may be supplied to the absorption column 100.

Water needed in the absorption column 100 may be supplied through the water supply line 5 provided in the upper portion of the cooling tower 20. The water may include, specifically, water such as tap water and deionized water, and may include circulation process water introduced from other processes (e.g., water phase recirculated from an extraction process and/or a distillation process). Further, the absorption solvent may include a small amount of organic by-products (e.g. acetic acid) introduced from other processes.

Meanwhile, most of the acetic acid included in the non-condensable gas by a contact with water in the cooling tower 20 is removed by dissolution in water, and gas which is not dissolved in water is discharged through a recirculation gas transfer line 4 provided in the upper portion of the cooling tower 20 as a recirculation gas. The recirculation gas may be supplied to the reactor 10 so that it may be used in the gas phase oxidation reaction for preparation of (meth)acrylic acid which proceeds in the reactor. The recirculation gas may be mixed with the reaction gas and supplied to the reactor, and may be supplied to the reactor through a separate line 4 from the line 1 to which the reaction gas is supplied.

Thereafter, a process for obtaining the (meth)acrylic acid aqueous solution by supplying the mixed gas 2 including (meth) acrylic acid which is the product of the gas phase oxidation reaction to the absorption column 100 through a reactor discharge line and bringing the gas into contact with water which is an absorption solvent in the absorption column 100 may be performed. Specifically, the mixed gas may include (meth)acrylic acid, organic by-products such as acetic acid and acrolein, and water vapor.

Herein, the kind of absorption column 100 may be determined considering contact efficiency of the mixed gas and the absorption solvent and the like, and for example, may be a packed column type absorption column or a multistage tray type absorption column. To the inside of the packed column type absorption column, a filler such as a Rasching ring, a Pall ring, a saddle, a gauze, and a structured packing may be applied.

Further, considering the efficiency of the absorption process, the mixed gas 2 may be supplied to the lower portion of the absorption column 100, and water which is the absorption solvent may be supplied to the upper portion of the absorption column 100.

Meanwhile, the absorption column 100 may be operated at an internal pressure of 1 to 1.5 bar or 1 to 1.3 bar and at an internal temperature of 50 to 100 °C or 50 to 90 °C, considering the condensation conditions of (meth)acrylic acid, a moisture content depending on a saturated water vapor pressure, and the like.

Meanwhile, according to an exemplary embodiment of the present invention, through an absorption process performed in the absorption column 100, the first (meth)acrylic acid aqueous solution discharged from the lower portion of the absorption column 100 and the second (meth)acrylic acid aqueous solution discharged from a side portion of the absorption column 100 may be obtained.

The lower portion of the absorption column 100 from which the first (meth)acrylic acid aqueous solution is discharged may be a point at a height of 95% to 100% from the top to the bottom of the absorption column 100, specifically, a bottom which is the lowest part of the absorption column 100. Meanwhile, the side portion of the absorption column 100 from which the second (meth)acrylic acid aqueous solution is discharged may be the side portion at a height of 40% to 80% from the top to the bottom of the absorption column 100. By setting a height (number of stages) at which the first and second (meth)acrylic acid aqueous solutions are discharged from the absorption column, contents of components included in the first and second (meth)acrylic acid aqueous solutions, for example, water and (meth)acrylic acid may be controlled.

The content of the (meth) acrylic acid in the first (meth)acrylic acid aqueous solution may be 75 wt% to 95 wt%, specifically 80 wt% to 90 wt%. In addition, a content of the water in the first (meth)acrylic acid aqueous solution may be 5 wt% to 20 wt%, specifically 10 wt% to 15 wt%. The first (meth)acrylic acid aqueous solution may include a residual amount of organic by-products in addition to the (meth)acrylic acid and water.

The content of the (meth) acrylic acid in the first (meth)acrylic acid aqueous solution is at a higher level than a content of the (meth) acrylic acid in the (meth) acrylic acid aqueous solution which is discharged from a conventional absorption column. In particular, by setting the content of the (meth)acrylic acid in the first (meth)acrylic acid aqueous solution to 75 wt% or more, the first (meth)acrylic acid aqueous solution may be directly supplied to the crystallizer 300 without undergoing a separate purification process or separation process for the first (meth)acrylic acid aqueous solution, and thus, overall process energy may be reduced, and also, high-purity (meth)acrylic acid may be obtained in the crystallizer 300.

The content of the (meth) acrylic acid in the second (meth)acrylic acid aqueous solution may be 30 wt% to 60 wt%, specifically 40 wt% to 55 wt%. In addition, a content of the water in the second (meth)acrylic acid aqueous solution may be 40 wt% to 60 wt%. The second (meth) acrylic acid aqueous solution may include a residual amount of organic by-products in addition to the (meth)acrylic acid and water. To this end, the second (meth)acrylic acid aqueous solution may be discharged from the side portion at a height of 40% to 80% from the top to the bottom of the absorption column 100.

Meanwhile, the upper discharge stream of the absorption column 100 may include a non-condensable gas which is not dissolved in water which is the absorption solvent in the absorption column 100, as described above, and the non-condensable gas may include acetic acid, inert gas, an unreacted raw material compound, and a minimal content of (meth)acrylic acid.

Conventionally, in order to obtain high-purity (meth)acrylic acid, it is common to discharge acetic acid as much as possible to the upper discharge stream of the absorption column. However, when the amount of acetic acid discharged to the upper portion of the absorption column 100 is increased and exceeds a certain level, the content of the (meth)acrylic acid discharged to the upper portion of the absorption column 100 is increased, and thus, the amount of (meth) acrylic acid lost in the absorption column is also increased. Therefore, the present invention may minimize the content of the (meth)acrylic acid lost in the absorption column, by controlling the amount of acetic acid included in the upper discharge stream of the absorption column 100. That is, the content of acetic acid in the upper discharge stream of the absorption column may be controlled so that the content of the (meth) acrylic acid lost to the upper portion of the absorption column may be minimized. In addition, since acetic acid may be further separated and removed by a water separation column and a layer separator after the absorption column described later, a problem that acetic acid accumulates in the system and acts as an impurity may be solved.

From this point of view, a ratio of a flow rate of acetic acid discharged to the upper portion of the absorption column may be 20 wt% to 80 wt%, specifically 30 wt% to 60 wt%, based on a flow rate of the acetic acid introduced to the absorption column. Meanwhile, the content of the (meth)acrylic acid included in the upper discharge stream of the absorption column may be 0.1 wt% to 0.5 wt%, specifically 0.2 wt% to 0.3 wt%.

Meanwhile, the first (meth)acrylic acid aqueous solution may be supplied to the crystallizer 300 through the lower discharge stream 130 of the absorption column, and the second (meth)acrylic acid aqueous solution may be introduced to a process of obtaining a distillate including (meth)acrylic acid and high-boiling point by-products through the side discharge stream 120 of the absorption column.

That is, by discharging the first and second (meth)acrylic acid aqueous solutions having different (meth)acrylic acid contents separately from the absorption column 100, energy usage consumed in a subsequent process may be reduced. Specifically, since the first (meth)acrylic acid aqueous solution has a high-concentration (meth)acrylic acid content, it may be directly supplied to the crystallizer 300 without undergoing a separate purification process or separation process for the first (meth)acrylic acid aqueous solution, and thus, overall process energy may be reduced, and also, high-purity (meth)acrylic acid may be obtained in the crystallizer 300. This is possible due to the high content of the (meth)acrylic acid in the first (meth)acrylic acid aqueous solution, as described above.

In addition, the first (meth)acrylic acid aqueous solution also includes a certain content of water. Since the mother liquor separated from the purified (meth)acrylic acid in the crystallizer 300 is circulated to the absorption column as described later, the amount of water included in the second (meth)acrylic acid aqueous solution is decreased by the amount of water included in the mother liquor, and thus, energy usage involved in the purification process for the second (meth)acrylic acid aqueous solution is reduced.

Specifically, the method for preparation of (meth)acrylic acid according to an exemplary embodiment of the present invention may include supplying the first (meth)acrylic acid aqueous solution to a crystallizer 300 and performing crystallization to obtain purified (meth)acrylic acid and a mother liquor separated from the purified (meth)acrylic acid. The (meth)acrylic acid which is crystallized in the crystallizer may be referred to as purified (meth)acrylic acid in some cases, in the present specification.

The (meth) acrylic acid included in the first (meth)acrylic acid aqueous solution supplied to the crystallizer 300 may be recrystallized through a crystallization process and obtained as purified high-purity (meth)acrylic acid. In addition, not only the first (meth)acrylic acid aqueous solution, but also an upper discharge stream of the high-boiling point by-product separation column which is discharged from an upper portion of a high-boiling point by-product separation column 600 described later and includes a high content of (meth)acrylic acid may be introduced to the crystallizer 300.

The crystallization process may be performed under common conditions.

The crystallization method for obtaining a product by crystallization in the present invention is not limited as to suspension crystallization and layer crystallization, may be continuous or batchwise, and may be performed in one stage or two stages or more. As a non-limited example, the (meth)acrylic acid may be dynamically crystallized and provided as purified (meth)acrylic acid.

Specifically, in order to dynamically crystallize the (meth)acrylic acid before crystallization, the (meth)acrylic acid aqueous solution may be first allowed to flow on a tube inner wall in a falling film form. Further, the temperature of the tube may be adjusted to a freezing point of (meth) acrylic acid or lower to form a crystal in the tube inner wall. Then, the temperature of the tube may be raised to near the freezing point of (meth) acrylic acid to sweat about 5 wt% of (meth)acrylic acid. Further, the mother liquor sweated from the tube is removed and the crystal formed in the tube inner wall is recovered, thereby obtaining the high-purity purified (meth)acrylic acid. The mother liquor may refer to a solution which is the (meth)acrylic acid aqueous solution introduced to the crystallizer 300 from which the purified (meth)acrylic acid has been removed. Therefore, the mother liquor may include (meth)acrylic acid, acetic acid, and high-boiling point by-products in addition to water, and (meth)acrylic acid herein may be residual (meth)acrylic acid which is not crystallized in the crystallizer 300.

Separation of the mother liquor and the crystallized purified (meth)acrylic acid may be performed using a solid-liquid separation device, for example, a belt filter, a centrifuge, and the like. The purified (meth)acrylic acid may be recovered as a (meth) acrylic acid recovery stream 310, and the mother liquor may be discharged from the crystallizer 300 through a mother liquor discharge stream.

According to an exemplary embodiment of the present invention, a part of the mother liquor discharged from the crystallizer 300 may be circulated to the absorption column 100 through a first mother liquor recovery line 320, and the rest of the mother liquor may be circulated to a water separation column 500 through a second mother liquor recovery line 330. Herein, the water separation column 500 is a device for obtaining a distillate including (meth)acrylic acid and high-boiling point by-products by being supplied with all or part of the second (meth) acrylic acid aqueous solution and distilling the solution, as described later.

Meanwhile, the mother liquor discharged from the crystallizer 300 may include 50 wt% to 80 wt%, specifically 60 wt% to 75 wt% of the (meth) acrylic acid. In addition, the mother liquor may include 20 wt% to 50 wt%, specifically 25 wt% to 40 wt% of the water. In addition, the mother liquor discharged from the crystallizer 300 may include a residual amount of organic by-products.

According to an exemplary embodiment of the present invention, the second (meth)acrylic acid aqueous solution may be introduced by a process of obtaining a distillate including (meth)acrylic acid and high-boiling point by-products.

Meanwhile, the process of obtaining a distillate including (meth)acrylic acid and high-boiling point by-products according to an exemplary embodiment of the present invention may be performed by including: supplying a part of the second (meth)acrylic acid aqueous solution to an extraction column 400 as an extraction column supply stream 170 and supplying the rest to a water separation column 500 as a water separation column supply stream 160; bringing an extraction solvent and the extraction column supply stream into contact in the extraction column 400 and supplying an extract stream including an extract to the water separation column 500; and separating an upper discharge stream 510 of the water separation column including water and the distillate including (meth)acrylic acid and high-boiling point by-products as a lower discharge stream 520 of the water separation column in the water separation column 500.

That is, the second (meth)acrylic acid aqueous solution was divided and supplied to the extraction column 400 and the water separation column 500, thereby reducing energy usage of a subsequent process and also achieving efficient separation of by-products such as acetic acid. Specifically, a ratio of a flow rate of the extraction column supply stream 170 supplied to the extraction column to a flow rate of the second (meth)acrylic acid aqueous solution may be 20 wt% to 60 wt%. When the flow rate ratio is 20 wt% or more, a flow rate introduced to the water separation column 500 is decreased and an energy amount consumed in distilling water having a high specific heat in the water separation column 500 may be decreased. Meanwhile, when the flow rate ratio is 60 wt% or less, acetic acid which is a by-product is efficiently separated to an upper portion of the water separation column 500 and the amount of acetic acid introduced to a high-boiling point by-product separation column 600 may be minimized. Thus, a content of acetic acid in an upper discharge stream of the high-boiling point by-product separation column 600 is lowered, and as a result, high-purity purified (meth)acrylic acid may be obtained in the crystallizer 300.

Meanwhile, the extraction column 400 removes most of water included in the extraction column supply stream 170 without use of large energy and supplies it to the water separation column 500, thereby saving energy used in azeotropic distillation in the water separation column 500 described later. In this respect, it is preferred that extraction in the extraction column 400 is performed by bringing an extraction solvent and the extraction column supply stream into contact by a liquid-liquid contact method in terms of improving energy efficiency of the entire process.

Herein, the extraction solvent may be a hydrocarbon solvent which forms an azeotrope with water and an organic by-products (acetic acid, etc.) and does not form an azeotrope with (meth)acrylic acid but may sufficiently extract (meth)acrylic acid, and also, it is advantageous in the extraction process that its boiling point is 10 to 120°C. Specifically, the extraction solvent may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

In addition, as the extraction column 400, an extraction device according to the liquid-liquid contact method may be used. A non-limiting example of the extraction device may include a Karr reciprocating plate column, a rotary-disk contactor, a Scheibel column, a Kuhni column, a spray extraction column, a packed extraction column, a pulsed packed column, a bank of mixer-settler, mixer and centrifuge (centrifugal counter current extractor), and the like.

In this way, a significant portion of water in the extraction column supply stream 170 supplied to the extraction column 400 is removed, an extract from which (meth)acrylic acid is extracted by the extraction solvent is obtained, and the extract may be supplied to the water separation column 500 as an extract stream 410. Specifically, the extract may include (meth)acrylic acid, acetic acid, the extract solvent, and high-boiling point by-products.

In addition, water included in the extraction column supply stream 170 through the extraction process may be recovered as a raffinate. The recovered raffinate may be discharged as a raffinate stream 420 and introduced to a layer separator 550 described later. Since water is recovered in the extraction process as such, the operation burden of the distillation process described later may be decreased to lower energy consumption.

Subsequently, according to an exemplary embodiment of the present invention, the water separation column supply stream 160 and the extract stream 410 in the second (meth)acrylic acid aqueous solution are supplied to the water separation column 500, and a distillation process for these streams may be performed.

A flow rate of water in the stream supplied to the water separation column 500 may be 30 wt% to 70 wt%, specifically 40 wt% to 60 wt%, based on the flow rate of the water introduced to the absorption column. When the flow rate ratio of water is less than 30 wt%, it may be difficult to separate acetic acid in the water separation column 500, and when the flow rate ratio is more than 70 wt%, the energy usage consumed for distilling water in the water separation column 500 and the high-boiling point by-product separation column 600 may be increased.

That is, by-products such as acetic acid in the water separation column 500 may be efficiently removed by controlling the flow rate and content of the water included in a stream supplied to the water separation column 500, and eventually, high-purity purified (meth)acrylic acid may be obtained. Simultaneously, energy usage required for distilling water in the water separation column 500 and the high-boiling point by-product separation column 600 described later may be reduced to the minimum, thereby reducing overall energy costs.

A distillation process in the water separation column 500 for the stream supplied to the water separation column 500 may be a process of separating an upper fraction including water, a hydrophobic azeotropic solvent, and acetic acid and a lower fraction including (meth)acrylic acid and high-boiling point by-products by azeotropic distillation.

According to the present invention, it is advantageous in the process that distillation in the water separation column 500 is performed in the presence of a hydrophobic azeotropic solvent, since the azeotropic solvent, water, and organic by-products (such as acetic acid) may be simultaneously recovered.

Herein, the hydrophobic azeotropic solvent is a hydrophobic solvent which may form an azeotrope with water and acetic acid and does not form an azeotrope with (meth)acrylic acid, and a hydrocarbon-based solvent satisfying the physical properties may be applied without limitation. Further, the hydrophobic azeotropic solvent may have a lower boiling point than (meth)acrylic acid, and may have a boiling point of preferably 10 to 120°C.

According to the present invention, the hydrophobic azeotropic solvent may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

Further, the hydrophobic azeotropic solvent may be the same as or different from the extraction solvent applied to the extraction column 400. However, considering production efficiency according to a continuous process and the like, it is preferred that the hydrophobic azeotropic solvent is the same as the extraction solvent. When the same compound is used as the azeotropic solvent and the extraction solvent as such, at least a part of the azeotropic solvent which is distilled in the water separation column 500 and recovered may be supplied to a lower portion of the extraction column 400 and used as a part of the extraction solvent.

When the hydrophobic azeotropic solvent is added to the water separation column 500 described above, an azeotrope of (meth)acrylic acid and water is broken. Accordingly, water, acetic acid, and the hydrophobic azeotropic solvent used in azeotropic distillation in the mother liquor may form an azeotrope together and be recovered as an upper fraction of the water separation column 500. Further, a lower fraction including (meth)acrylic acid and high-boiling point by-products may be recovered to a lower portion of the water separation column 500.

The upper fraction of the water separation column recovered as such may be supplied to a layer separator 550 through an upper discharge stream 510 of the water separation column, and a lower fraction of the water separation column may be supplied to the high-boiling point by-product separation column 600 through a lower discharge stream 520 of the water separation column.

Herein, the layer separator 550 is a liquid-liquid layer separator and a device for separating fluids which are not mixed with each other by a density difference using gravity, centrifugal force, or the like, in which a relatively light liquid may be separated to the upper portion of the layer separator 550 and a relatively heavy liquid may be separated to the lower portion of the layer separator 550. Specifically, the upper discharge stream 510 of the water separation column supplied to the layer separator 550 may be separated into an organic layer including a hydrophobic azeotropic solvent and a water layer including water and acetic acid.

In addition, the organic layer separated from the layer separator 550 is discharged as a discharge stream 560 of the layer separator, a part of the discharge stream 560 of the layer separator may be supplied to the upper portion of the water separation column 500 and reused as an azeotropic solvent, and the rest may be supplied to the extraction column 400 and reused as the extraction solvent.

Meanwhile, in the layer separator 550, a part of the water layer including water and acetic acid may be supplied to the upper portion of the absorption column 100 and used as an absorption solvent, and the rest may be discharged as waste water.

Herein, acetic acid may be included in the water layer, and a concentration of acetic acid included in the water layer may vary depending on the kind of azeotropic solvent, a reflux ratio of a column installed in the water separation column 500, and the like. According to the present invention, the concentration of acetic acid included in the water layer may be 1 to 30 wt%, preferably 2 to 20 wt%, and more preferably 3 to 10 wt%.

That is, according to an exemplary embodiment of the present invention, acetic acid may be discharged through the upper discharge stream of the absorption column 100, and also discharged by azeotropic distillation performed through the water separation column 500 and the layer separator 550. Therefore, acetic acid accumulating in the system may be more efficiently removed than a process of removing acetic acid only in the upper portion of the absorption column, thereby obtaining high-purity purified (meth)acrylic acid therefrom. Furthermore, process flexibility may be secured as compared with an attempt to discharge a total amount of acetic acid in the system to the upper portion of the absorption column, and this has an advantage of minimizing a loss of (meth)acrylic acid from the upper portion of the absorption column.

From this point of view, a total flow rate of acetic acid introduced to the absorption column 100 may be the same as the sum of a flow rate of acetic acid in the upper discharge stream of the absorption column 100 and a flow rate of acetic acid in a stream which is included water layer of the layer separator 550 and is discharged.

Meanwhile, the method for preparation of (meth)acrylic acid according to an exemplary embodiment of the present invention may include supplying the lower discharge stream 520 of the water separation column 500 to the high-boiling point by-product separation column 600 and supplying the upper discharge stream of the high-boiling point by-product separation column 600 including (meth)acrylic acid to the crystallizer 300.

The lower discharge stream of the water separation column 500 may be distilled by the high-boiling point by-product separation column 600 to be separated into a lower fraction including high-boiling point by-products and an upper fraction including a high content of (meth)acrylic acid by removing the high-boiling point by-products. The upper fraction may be supplied to the crystallizer 300 through the upper discharge stream 610 of the high-boiling point by-product separation column, and the content of the (meth)acrylic acid included in the upper discharge stream 510 of the high-boiling point by-product separation column may be 90 wt% to 99 wt%, specifically 95 wt% to 99 wt%.

That is, since the amount water included in each stream is already been reduced in the process after the absorption column 100 and also a significantly amount water is removed through the extraction column 400 and the water separation column 500, the content of the water in the upper discharge stream 610 of the high-boiling point by-product separation column is controlled to be low, and thus, a highly concentrated stream of (meth)acrylic acid which may be directly introduced to the crystallizer 300 may be implemented. The loss of (meth) acrylic acid may be decreased to the maximum by introducing the upper discharge stream 610 of the high-boiling point by-product separation column to the crystallizer 300.

Meanwhile, the first (meth)acrylic acid aqueous solution discharged from the lower portion of the absorption column and the upper discharge stream of the high-boiling point by-product separation column may be supplied to the crystallizer 300 as separate streams, respectively, and also, these streams may form a mixed stream and be supplied to the crystallizer 300.

The content of the (meth)acrylic acid included in the upper discharge stream of the high-boiling point by-product separation column may be higher than the content of the (meth)acrylic acid included in the first (meth)acrylic acid aqueous solution, and when the first (meth)acrylic acid aqueous solution discharged from the lower portion of the absorption column and the upper discharge stream of the high-boiling point by-product separation column form a mixed stream and are supplied to the crystallizer, the content of the (meth)acrylic acid in the mixed stream may be 85 to 99 wt%. Therefore, the mixed stream may be directly introduced to the crystallizer without a separate distillation process.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

### Example 1

According to the process flow illustrated in FIG. 1, a preparation process of (meth)acrylic acid was simulated, using an Aspen Plus simulator available from Aspen.

Specifically, a reaction gas including air and a raw material compound (propylene) was supplied to a reactor 10 provided with a catalyst through a reaction gas supply line 1, and a recirculation gas derived from a cooling tower 20 was supplied to the reactor 10 through a recirculation gas transfer line 4. A mixed gas 2 having a composition including (meth)acrylic acid (6.6 mol%), water (16.4 mol%), a high-boiling point material (0.09 mol%), and inert gas (76.3 mol%) was obtained by a gas phase oxidation reaction performed in the reactor 10.

The mixed gas 2 was added to a 11th stage from the top of an absorption column 100 at a temperature of 164°C. In the absorption column 100, the mixed gas was brought into contact with an absorption solvent (water) to obtain a first (meth)acrylic acid aqueous solution and a second (meth)acrylic acid aqueous solution. The water added to the absorption column 100 was supplied through a lower discharge stream 6 of the cooling tower and a water layer from a layer separator 550 described later, and the water was supplied to the upper portion of the absorption column 100 at a flow rate of 10.6 wt% to the mass flow rate of the mixed gas 2. At this time, the pressure of the upper portion of the absorption column 100 was 1.1 bar, and a temperature of the lower portion of the absorption column 100 was 82 °C.

Meanwhile, in the absorption column 100, a non-condensable gas including components which were not dissolved in water was separated as the upper discharge stream 110 of the absorption column, and a part 3 of the upper discharge stream of the absorption column was supplied to the cooling tower 20 and the rest was discharged out of the system.

In the cooling tower 20, the non-condensable gas included in the part 3 of the upper discharge stream of the absorption column was dissolved in water. At this time, the water was supplied through a water supply line 5. In the cooling tower 20, the gas which was not dissolved in water was supplied to the reactor 10 through a recirculation gas transfer line 4, and the lower discharge stream 6 of the cooling tower including water and components dissolved in water (acetic acid and (meth) acrylic acid which was not dissolved in water in the absorption column) was supplied to the upper portion of the absorption column 100.

Meanwhile, the first (meth)acrylic acid aqueous solution described above included (meth)acrylic acid (79.2 wt%), acetic acid (2.2 wt%), water (17 wt%), furfural (0.7 wt%), and maleic acid (0.8 wt%), and was supplied to the crystallizer 300 as the lower discharge stream 130 of the absorption column. At this time, the lower discharge stream 130 of the absorption column formed a mixed stream with the upper discharge stream 610 of the high-boiling point by-product separation column and was supplied to the crystallizer 300. The content of the (meth)acrylic acid in the mixed stream was 88.6 wt%.

A (meth)acrylic acid recovery stream 310 including (meth)acrylic acid was obtained by a crystallization process performed in the crystallizer 300, and the content of the (meth)acrylic acid in the (meth)acrylic acid recovery stream 310 was 99.5 wt% or more. Finally, 99.5 wt% or more of (meth)acrylic acid was obtained from the crystallizer 300.

In addition, a mother liquor separated from the (meth)acrylic acid was obtained in the crystallizer 300. At this time, the mother liquor included (meth)acrylic acid (63.2 wt%), water (25.3 wt%), acetic acid (3.9 wt%), furfural (3.1 wt%), and maleic acid (4.5 wt%). The mother liquor was branched, and was supplied to the absorption column 100 and the water separation column 500 through a first mother liquor recovery line 320 and a second mother liquor recovery line 330, respectively.

Meanwhile, the second (meth)acrylic acid aqueous solution described above included (meth)acrylic acid (43.8 wt%), acetic acid (4.3 wt%), water (50.9 wt%), furfural (0.4 wt%), and maleic acid (0.03 wt%), and was supplied to the extraction column 400 and the water separation column 500 as a side discharge stream 120 of the absorption column at a height of 64% from the top to the bottom of the absorption column 100. At this time, the side discharge stream 120 of the absorption column including the second (meth)acrylic acid aqueous solution was branched, and 45 wt% of the total weight of the second (meth)acrylic acid aqueous solution was supplied to the extraction column 400 as an extraction column supply stream 170 and the remaining 55 wt% of the second (meth) acrylic acid aqueous solution was supplied to the water separation column 500 as a water separation column supply stream 160. Meanwhile, the mass flow rate of the side discharge stream 120 of the absorption column was 0.77 times the mass flow rate of the lower discharge stream 130 of the absorption column.

A raffinate stream 420 including water and the extract stream 410 including toluene and (meth)acrylic acid were obtained, by an extraction process performed in the presence of an extraction solvent (toluene) in the extraction column 400. At this time, the extraction solvent (toluene) supplied to the lower portion of the extraction column 400 was supplied at a flow rate of 4.32 times the mass flow rate of water in the second (meth)acrylic acid aqueous solution supplied to the extraction column 400. The raffinate stream 420 was supplied to a layer separator 550 described later, and the extract stream 410 was mixed with the water separation column supply stream 160 described above and supplied to the water separation column 500.

In the water separation column 500, an upper discharge stream 510 of the water separation column including toluene, water, and acetic acid and a lower discharge stream 520 of the water separation column including (meth)acrylic acid and high-boiling point by-products were obtained, by azeotropic distillation performed in the presence of a hydrophobic azeotropic solvent (toluene). At this time, the hydrophobic azeotropic solvent supplied to the water separation column 500 was supplied at a flow rate of 1.5 times the mass flow rate of the extraction solvent supplied to the extraction column 400.

The upper discharge stream 510 of the water separation column was supplied to a layer separator 550 and separated into a water layer 570 including water and acetic acid and an organic layer 560 including toluene. A part of the water layer was supplied to the absorption column 100 and the rest was discharged out of the system as waste water. Meanwhile, the organic layer was supplied to the extraction column 400 and the water separation column 500.

Meanwhile, the lower discharge stream 520 of the water separation column described above was supplied to a high-boiling point by-product separation column 600 to obtain an upper discharge stream 610 of the high-boiling point by-product separation column including (meth)acrylic acid and a lower discharge stream 620 of the high-boiling point by-product separation column including high-boiling point by-products. The upper discharge stream 610 of the high-boiling point by-product separation column was mixed with a lower discharge stream 130 of the absorption column and supplied to the crystallizer 300, as described above. At this time, the content of the (meth)acrylic acid in the upper discharge stream 610 of the high-boiling point by-product separation column was 99.2 wt%%.

As a result, energy used in the water separation column 500 was 369.1 kcal/kg AA, energy used in the high-boiling point by-product separation column 600 was 82.3 kcal/kg AA, energy used in the crystallizer 300 was 130.5 kcal/kg AA, and thus, a total of 581.8 kcal/kg AA of energy was used.

### Comparative Example 1

According to the process flow illustrated in FIG. 2, a preparation process of (meth)acrylic acid was simulated, using the Aspen Plus simulator available from Aspen.

Specifically, in Comparative Example 1, a reaction product 2 obtained by the same process as in Example 1 was added to the absorption column under the same conditions (operating conditions and flow rate of water added to the absorption column), but the (meth)acrylic acid aqueous solution was not obtained from the side portion and the bottom of the absorption column 100, but obtained only from the bottom of the absorption column.

The (meth)acrylic acid aqueous solution was supplied to the degassing column 150 to obtain an upper discharge stream of the degassing column including low-boiling point by-products and a lower discharge stream 160 of the degassing column including the (meth)acrylic acid aqueous solution from which the low-boiling point by-products had been removed. At this time, the upper discharge stream from the degassing column had a flow rate of 13.6 wt% as compared with the mass flow rate of water added to the absorption column, and the lower discharge stream 160 of the degassing column had a flow rate of 2.3 times the mass flow rate of water added to the absorption column. The lower discharge stream 160 of the degassing column included (meth)acrylic acid (64.3 wt%), acetic acid (2.7 wt%), water (31.9 wt%), furfural (0.5 wt%), and maleic acid (0.6 wt%).

The lower discharge stream 160 of the degassing column was supplied to the water separation column 500, and azeotropically distilled in the presence of a hydrophobic azeotropic solvent (toluene) to obtain an upper discharge stream 510 of the water separation column including toluene, water, and acetic acid and a lower discharge stream 520 of the water separation column including (meth)acrylic acid and high-boiling point by-products. At this time, the hydrophobic azeotropic solvent (toluene) was supplied to the upper portion of the water separation column 500 at a flow rate of 2.1 times the flow rate of the lower discharge stream 160 of the degassing column.

The upper discharge stream 510 of the water separation column was supplied to a layer separator 550 to obtain a water layer 570 including water and acetic acid and an organic layer 560 including toluene. A part of the water layer 570 was supplied to the absorption column 100 and the rest was discharged out of the system as waste water. Meanwhile, the organic layer 560 was circulated to the water separation column 500.

Meanwhile, the lower discharge stream 520 of the water separation column was supplied to a high-boiling point by-product separation column 600 to obtain a lower discharge stream 620 of the high-boiling point by-product separation column including high-boiling point by-products and an upper discharge stream 610 of the high-boiling point by-product separation column including (meth)acrylic acid. The content of the (meth)acrylic acid in the upper discharge stream 610 of the high-boiling point by-product separation column was 99.5 wt% or more, and finally, 99.5 wt% or more (meth)acrylic acid was obtained from the high-boiling point by-product separation column 600.

As a result, energy used in the water separation column 500 was 683.6 kcal/kg AA and energy used in the high-boiling point by-product separation column 600 was 179.5 kcal/kg AA, and thus, a total of 863.1 kcal/kg AA of energy was used.

### Comparative Example 2

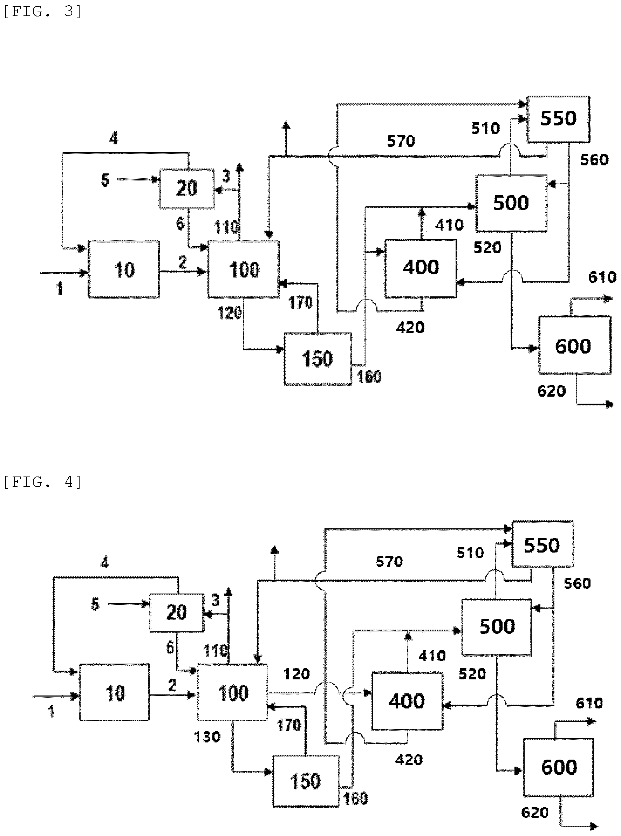

According to the process flow illustrated in FIG. 3, a preparation process of (meth)acrylic acid was simulated, using the Aspen Plus simulator available from Aspen.

In Comparative Example 2, the lower discharge stream 160 of the degassing column having the same composition as Comparative Example 1 was branched at 35 wt% and 65 wt%, respectively, of the total weight of the lower discharge stream 160 of the degassing column, and supplied to the extraction column 400 and the water separation column 500, respectively.

A raffinate stream 420 including water and the extract stream 410 including toluene and (meth)acrylic acid were obtained, by an extraction process performed in the presence of an extraction solvent (toluene) in the extraction column 400. At this time, the extraction solvent was supplied to the lower portion of the extraction column 400 at a flow rate of 4.1 times the mass flow rate of water in the lower discharge stream 1260 of the degassing column which was branched to the upper portion of the extraction column 400 and supplied. The raffinate stream 420 was supplied to the layer separator 550, and the extract stream 410 was mixed with the lower discharge stream of the degassing column which was branched to the water separation column 500 and supplied to the water separation column 500.

In the water separation column 500, an upper discharge stream 510 of the water separation column including toluene, water, and acetic acid and a lower discharge stream 520 of the water separation column including (meth)acrylic acid and high-boiling point by-products were obtained, by azeotropic distillation performed in the presence of a hydrophobic azeotropic solvent (toluene). At this time, the hydrophobic azeotropic solvent was supplied to the upper portion of the water separation column 500 at a mass flow rate of 2.1 times the mass flow rate of the extraction solvent supplied to the extraction column 400.

The upper discharge stream 510 of the water separation column was supplied to a layer separator 550 to obtain a water layer 570 including water and acetic acid and an organic layer 560 including toluene. A part of the water layer was supplied to the absorption column 100 and the rest was discharged out of the system as waste water. Meanwhile, the organic layer 560 was supplied to the water separation column 500 and the extraction column 400.

Meanwhile, the lower discharge stream 520 of the water separation column was supplied to a high-boiling point by-product separation column 600 to obtain a lower discharge stream 620 of the high-boiling point by-product separation column including high-boiling point by-products and an upper discharge stream 610 of the high-boiling point by-product separation column including (meth)acrylic acid. The content of the (meth)acrylic acid in the upper discharge stream 610 of the high-boiling point by-product separation column was 99.5 wt% or more, and finally, 99.5 wt% or more (meth)acrylic acid was obtained from the high-boiling point by-product separation column 600.

As a result, energy used in the water separation column 500 was 474.5 kcal/kg AA and energy used in the high-boiling point by-product separation column 600 was 179.5 kcal/kg AA, and thus, a total of 654 kcal/kg AA of energy was used.

### Comparative Example 3

According to the process flow illustrated in FIG. 4, a preparation process of (meth)acrylic acid was simulated, using the Aspen Plus simulator available from Aspen.

Specifically, in Comparative Example 3, the flow rate of the second (meth)acrylic acid aqueous solution discharged to the side portion of the absorption column was 0.23 times the mass flow rate of the first (meth)acrylic acid aqueous solution discharged to the bottom of the absorption column.

The first (meth)acrylic acid aqueous solution was supplied to the degassing column 150 as the lower discharge stream 130 of the absorption column. The lower discharge stream 130 of the absorption column was degassed in the degassing column 150, and low-boiling point by-products were supplied to the absorption column 100 as an upper discharge stream 170 of the degassing column and a (meth)acrylic acid aqueous solution from which the low-boiling point by-products had been degassed was supplied to the water separation column 500 as a lower discharge stream 160 of the degassing column. At this time, the lower discharge stream 160 of the degassing column included (meth)acrylic acid (73 wt%), acetic acid (2.4 wt%), water (23.3 wt%), furfural (0.6 wt%), and maleic acid (0.7 wt%).

Meanwhile, the second (meth)acrylic acid aqueous solution was supplied to the extraction column 400 as the side discharge stream 120 of the absorption column. The second (meth)acrylic acid aqueous solution included (meth)acrylic acid (26.4 wt%), acetic acid (4.1 wt%), water (68.8 wt%), furfural (0.14 wt%), and maleic acid (0.03 wt%).

A raffinate stream 420 including water and the extract stream 410 including toluene and (meth)acrylic acid were obtained, by an extraction process performed in the presence of an extraction solvent (toluene) in the extraction column 400. At this time, the extraction solvent was supplied to the lower portion of the extraction column at a flow rate of 3.35 times the mass flow rate of water in the second (meth)acrylic acid aqueous solution supplied to the upper portion of the extraction column. The raffinate stream 420 was supplied to a layer separator 550, and the extract stream 410 was supplied to the water separation column 500. At this time, the extract stream 410 was mixed with the lower discharge stream 160 of the degassing column described above and supplied to the water separation column 500 as a mixed stream.

In the water separation column 500, an upper discharge stream 510 of the water separation column including toluene, water, and acetic acid and a lower discharge stream 520 of the water separation column including (meth)acrylic acid and high-boiling point by-products were obtained, by azeotropic distillation performed in the presence of a hydrophobic azeotropic solvent (toluene). At this time, the hydrophobic azeotropic solvent was supplied to the upper portion of the water separation column 500 at a mass flow rate of 2.45 times the mass flow rate of the extraction solvent supplied to the extraction column 400 described above.

The upper discharge stream 510 of the water separation column was supplied to a layer separator 550 to obtain a water layer including water and acetic acid and an organic layer 560 including toluene. A part of the water layer was supplied to the absorption column 100 and the rest was discharged out of the system as waste water. Meanwhile, the organic layer was circulated to the water separation column 500 and the extraction column 400.

Meanwhile, the lower discharge stream 520 of the water separation column was supplied to a high-boiling point by-product separation column 600 to obtain a lower discharge stream 620 of the high-boiling point by-product separation column including high-boiling point by-products and an upper discharge stream 610 of the high-boiling point by-product separation column including (meth)acrylic acid. The content of the (meth)acrylic acid in the upper discharge stream 610 of the high-boiling point by-product separation column was 99.5 wt% or more, and finally, 99.5 wt% or more (meth)acrylic acid was obtained from the high-boiling point by-product separation column 600.

As a result, energy used in the water separation column 500 was 455.9 kcal/kg AA and energy used in the high-boiling point by-product separation column 600 was 179.5 kcal/kg AA, and thus, a total of 635.5 kcal/kg AA of energy was used.

In Comparative Example 3, it was confirmed that though the lower discharge stream of the water separation column was not introduced to the crystallizer as in Comparative Examples 1 and 2, 99.5 wt% or more of (meth)acrylic acid was able to be obtained through the high-boiling point by-product separation column, but total energy usage was increased as compared with Example 1 further provided with the crystallizer.

## Claims

1. A method for preparation of (meth)acrylic acid, the method comprising:
bringing a mixed gas including (meth)acrylic acid into contact with water in an absorption column to obtain first and second (meth)acrylic acid aqueous solutions;
discharging the first (meth)acrylic acid aqueous solution from a lower portion of the absorption column and supplying the solution to a crystallizer, and discharging the second (meth)acrylic acid aqueous solution from a side portion of the absorption column;
supplying the second (meth)acrylic acid aqueous solution to a water separation column to obtain a distillate including (meth)acrylic acid and high-boiling point by-products;
supplying the distillate to a high-boiling point by-product separation column and supplying an upper discharge stream of the high-boiling point by-product separation column including (meth)acrylic acid to the crystallizer; and
obtaining purified (meth)acrylic acid in the crystallizer and circulating a part of a mother liquor recovered in the crystallizer to the absorption column and the rest to the water separation column.

2. The method for preparation of (meth)acrylic acid of claim 1, wherein a content of the (meth)acrylic acid in the first (meth) acrylic acid aqueous solution is 75 wt% to 95 wt%, and a content of the (meth)acrylic acid in the second (meth)acrylic acid aqueous solution is 30 wt% to 60 wt%.

3. The method for preparation of (meth)acrylic acid of claim 1, wherein the second (meth)acrylic acid aqueous solution is discharged from the side portion at a height of 40% to 80% from the top to the bottom of the absorption column.

4. The method for preparation of (meth)acrylic acid of claim 1, wherein a content of the (meth)acrylic acid in an upper discharge stream of the high-boiling point by-product separation column is 90 wt% to 99 wt%.

5. The method for preparation of (meth)acrylic acid of claim 1,
wherein the first (meth)acrylic acid aqueous solution discharged from the lower portion of the absorption column and the upper discharge stream of the high-boiling point by-product separation column form a mixed stream and are supplied to the crystallizer, and
a content of the (meth)acrylic acid in the mixed stream is 85 to 99 wt%.

6. The method for preparation of (meth)acrylic acid of claim 1, wherein a flow rate ratio of acetic acid discharged to an upper portion of the absorption column is 20 wt% to 80 wt%, based on a flow rate of the acetic acid introduced to the absorption column.

7. The method for preparation of (meth)acrylic acid of claim 1, wherein the obtaining of a distillate including (meth)acrylic acid and high-boiling point by-products includes:
supplying a part of the second (meth)acrylic acid aqueous solution to an extraction column as an extraction column supply stream and supplying the rest to a water separation column as a water separation column supply stream;
bringing an extraction solvent and the extraction column supply stream into contact in the extraction column and supplying an extract stream including an extract to the water separation column; and
separating an upper discharge stream of the water separation column including water and the distillate including (meth)acrylic acid and high-boiling point by-products as a lower discharge stream of the water separation column in the water separation column.

8. The method for preparation of (meth)acrylic acid of claim 7, wherein a ratio of a flow rate of the extraction column supply stream supplied to the extraction column to a flow rate of the second (meth)acrylic acid aqueous solution is 20 wt% to 60 wt%.

9. The method for preparation of (meth)acrylic acid of claim 7, wherein the upper discharge stream of the water separation column is supplied to a layer separator, a part of a water layer including water and acetic acid is circulated to the absorption column, and the rest is discharged as waste water.

10. The method for preparation of (meth)acrylic acid of claim 7, wherein the extraction solvent and the extraction column supply stream are brought into contact in the extraction column to further obtain a raffinate stream including a raffinate, and the raffinate stream is supplied to the layer separator.
